# EUROPEAN PATENT SPECIFICATION

(11) **EP 3 733 108 B1**
(45) Date of publication and mention of the grant of the patent: **23.08.2023**
(21) Application number: 18893614.0
(22) Date of filing: 12.11.2018
(51) Int. Cl.: A61B 34/30, B25J 9/10, B25J 18/00, B25J 9/16, B25J 9/04, B25J 9/00, A61B 90/50

(54) **SURGICAL ROBOT TERMINAL**
CHIRURGISCHER ROBOTERANSCHLUSS
TERMINAL DE ROBOT CHIRURGICAL

(30) Priority: 27.12.2017 CN 201711445629
(43) Date of publication of application: 04.11.2020
(73) Proprietor: Shanghai Microport Medbot (Group) Co., Ltd., Shanghai 201203 (CN)
(72) Inventor: LI, Tao, Shanghai 201203 (CN); HE, Chao, Shanghai 201203 (CN); XIA, Yuhui, Shanghai 201203 (CN); JIANG, Youkun, Shanghai 201203 (CN)
(74) Representative: Patentship Patentanwaltsgesellschaft mbH
(86) International application number: PCT/CN2018/114943
(87) International publication number: WO 2019/128494

(56) References cited:
- WO-A1-2006/079108
- CN-A- 102 973 317
- CN-A- 106 236 267
- CN-A- 106 236 276
- CN-A- 106 618 736
- CN-A- 107 374 736
- CN-A- 107 374 736
- CN-A- 108 056 823
- CN-A- 108 186 120
- US-A1- 2004 261 179
- US-A1- 2014 257 328
- US-A1- 2017 079 722

## Description

### TECHNICAL FIELD

The present application relates to the field of medical devices and, more specifically, to a surgical robot terminal.

### BACKGROUND

Because of the advantages including smaller wound and rapid recovery, minimally invasive surgery becomes more and more popular among patients. More and more surgeries change their operation forms from traditional open operation to minimally invasive operation. At the same time, surgical tools are also gradually evolving from the early forceps and scalpels to the contemporary surgical robotic arms, surgical robots and the like.

The rapid development of robotics is continuously bringing about the emergence of a wide variety of robots with various features, from the early single-armed ones to the recent ones with two or more coordinated arms. Such a multi-arm robot has master-slave operations to better substitute the surgeon's hands, in which the salve is a terminal of the robot with multi-arm. While these arms can be used to perform a wide range of surgeries, the structure of them can have a direct impact on the performance of the surgical robot's terminal.

Differing from traditional industrial robots, those for medical use must ensure good human-machine collaboration, i.e., a high degree of consistency of the robotic arms' movements with the surgeon's hand movements during a practical operation. To this end, the robot must be accurately adjusted in position relative to the patient in order to ensure a desirable spatial relationship therebetween before the operation is carried out.

In order to meet this requirement, there have been proposed a number of surgical robots with terminal arrangements allowing such positional adjustments, which, however, suffer from the following deficiencies:
(1) Limited Adjustability of Robotic Arms - Pat. Pub. No. CN102579133A proposes a terminal of a surgical robot, comprising a suspension disc arranged above robotic arms in order to adjust the overall robotic arms to an orientation suitable for access to a target surgical site. However, this solution tends to hang the suspension disc on the ceiling of an operating room, necessitating adjusting a surgical bed to ensure better access of the robotic arms to the target surgical site in the patient lying in the bed.
(2) Limited Adjustment Ranges of Robotic arms - When fixed to the surgical bed, a robotic arm will have a significantly limited movement range. Likewise, the robotic arms having a fixed relative positional relationship with the others will possess limited movability. The surgical robot with an overhead suspension structure allows overall adjustments of robotic arms, but it causes the fixed relative positions of robotic arms and the constrained adjustment ranges, limiting the overall adjustability of the terminal.
(3) Easy Interference between Robotic arms - As an improvement over the above solutions, Pat. Pub. No. CN104334112A proposes a solution in which suspended robotic arms are arranged on a movable base and can thus move in a wider range. However, as these robotic arms each have multiple degrees of freedom and can move relative to one another, when they are coupled to a single suspension disc, there will be limited spaces between adjacent robotic arms for them to avoid from colliding with each other. Thus, in this case, interference between robotic arms that may hinder the implementation of a surgical operation tends to take place.

US 2017/079722 A1 discloses methods and systems for registering a manipulator assembly and independently positionable surgical table, wherein the methods include reading a fiducial marker on the surgical table with a sensor associated with the manipulator assembly and localizing the manipulator assembly and surgical table with respect to a common reference frame, wherein the methods further include translating a 3D configuration of the surgical table to a 2D frame of reference so as to estimate a 3D pose of the surgical table relative the manipulator assembly for use in coordinating movements therebetween. CN 107 374 736 A discloses a surgical cart comprising a standing column, wherein vertical guide rails are arranged on the outer walls of the standing column and a slider is slidably arranged on each vertical guide rail, wherein a base for mounting a mechanical arm is fixedly arranged on each slider, and limit devices for controlling the corresponding base to move in stroke limit are respectively arranged at the top and the bottom of each outer wall of the standing column. CN 106 236 276 A discloses a surgical robot system comprising robotic arms, a base, a support connected to the base, and a suspension structure connected to the support, wherein the suspension structure is connected to the robotic arms. WO 2006/079108 A1 discloses a robotic surgical system comprising a manipulator support assembly, wherein the manipulator support assembly comprises an orienting platform and a plurality of configurable set-up joint arms couplable to the orienting platform. US 2004/261179 A1 discloses a linkage structure rotatably connected to the ceiling. CN 106 618 736 A discloses a robotic arm comprising a support structure that functions as a support for the robotic arm.

### SUMMARY OF THE INVENTION

The invention is set out in the appended set of claims.

It is an objective of the some embodiments to provide a terminal of a surgical robot that is free of at least one of the problems associated with such conventional terminal.

To solve the above problem, the present application provides a terminal a surgical robot terminal, comprising:
a movable base;
a support mechanism comprising at least two arm support;
a vertical assembly having a base end fixedly connected to the base and a support end located above the base and rotatably coupled to the support mechanism; and
a plurality of robotic arms, each comprising a setup arm and a tool arm, the setup arm having a proximal end coupled to one of the at least two arm supports and a distal end coupled to the tool arm, the tool arm having a distal end coupled to a surgical instrument, the tool arm being configured to drive the surgical instrument to pivot about a remote center of motion, the setup arm being configured to adjust a spatial position of the remote center of motion,
wherein the vertical assembly comprises a vertical translational joint configured to adjust a height of the remote center of motion relative to the base, and
wherein the base comprises at least two wheel sets, each comprising a drive wheel and a guide wheel, the guide wheel configured for control the movement direction, the drive wheel configured for control the movement speed, the at least two wheel sets configured to cooperate to change the position of the base so as to change a horizontal position of the remote center of motion.

In the surgical robot terminal, each of the arm supports has a proximal end coupled to the support end of the vertical assembly and a distal end coupled to at least one of the plurality of setup arms.

Optionally, in the surgical robot, the support mechanism further comprises a primary arm support having a proximal end rotatably coupled to the support end of the vertical assembly, and wherein each of the at least two arm supports has a proximal end rotatably coupled to a distal end of the primary arm support and a distal end coupled to at least one of the plurality of setup arms.

Optionally, in the surgical robot terminal, each of the plurality of setup arms comprises a first rotary joint, a first horizontal translational joint, a swinging joint and a second rotary joint, which are sequentially coupled together successively,
the first horizontal translational joint having an axis of translation that is perpendicular to an axis of rotation of the first rotary joint,
the swinging joint having an axis of rotation that is perpendicular to both the axis of translation of the first horizontal translational joint and the axis of rotation of the first rotary joint,
the second rotary joint having an axis of rotation oriented parallel to the axis of rotation of the first rotary joint,
the first rotary joint coupling the corresponding setup arm to the support mechanism, the second rotary joint coupling the corresponding setup arm to the corresponding tool arm.

Optionally, in the surgical robot terminal, the swinging joint comprises a first parallelogram structure formed by articulated links including a first proximal link and a first distal link parallel to the first proximal link, the first proximal link extending parallel to the axis of rotation of the first rotary joint, the first distal link being coupled to the second rotary joint and extending parallel to the axis of rotation of the second rotary joint.

Optionally, in the surgical robot terminal, each of the plurality of setup arms comprises a connecting rod rotatably coupled to both the swinging joint and the second rotary joint, a measurement component for measuring a swing angle of the swinging joint and a motor for driving the second rotary joint to swing relative to the connecting rod,
the measurement component being communicatively coupled to the motor that is configured to drive the second rotary joint to swing in accordance with the swing angle of the swinging joint received from the measurement component so that the axis of rotation of the second rotary joint is maintained parallel to the axis of rotation of the first rotary joint.

Optionally, in the surgical robot terminal, each of the plurality of setup arms further comprises a second horizontal translational joint having a proximal end coupled to the support mechanism and a distal end coupled to the first rotary joint, the second horizontal translational joint having an axis of translation that is perpendicular to the axis of rotation of the first rotary joint.

According to the invention, in the surgical robot terminal, each of the plurality of setup arms comprises a first horizontal translational joint, a first rotary joint, a swinging joint and a second rotary joint, which are sequentially coupled together successively,
the first horizontal translational joint having an axis of translation that is perpendicular to an axis of rotation of the first rotary joint,
the swinging joint having an axis of rotation that is perpendicular to both the axis of translation of the first horizontal translational joint and the axis of rotation of the first rotary joint,
the second rotary joint having an axis of rotation oriented parallel to the axis of rotation of the first rotary joint,
the first horizontal translational joint coupling the corresponding setup arm to the support mechanism, the second rotary joint coupling the corresponding setup arm to the corresponding tool arm.

Optionally, in the surgical robot terminal, each of the plurality of tool arms has three or more degrees of freedom, and thus is able to drive the surgical instrument to at least swing left and right, pivot front and back and move up and down with respect to the remote center of motion.

Optionally, in the surgical robot terminal, each of the plurality of tool arms comprises a base joint, a telescopic joint, and a second parallelogram structure formed by articulated links,
the base joint being configured to swing about a first axis so as to drive the surgical instrument to swing about the first axis, the base joint having a proximal end coupled to the setup arm and a distal end coupled to the second parallelogram structure,
the second parallelogram structure comprising a second proximal link and a second distal link parallel to the second proximal link, the second parallelogram structure configured to drive the surgical instrument to pivot about a second axis,
the telescopic joint being coupled to the second distal link, the telescopic joint having an axis of translation that is parallel to an axis of the second distal link, the telescopic joint being detachably coupled to the surgical instrument so as to be able to drive the surgical instrument to translate along the axis of translation of the telescopic joint,
wherein the remote center of motion is defined at an intersection of the first axis, the second axis and the axis of translation of the telescopic joint.

Optionally, in the surgical robot terminal, each of the plurality of tool arms has two or more degrees of freedom, and thus is able to drive the surgical instrument to at least swing left and right and pivot front and back with respect to the remote center of motion.

Optionally, in the surgical robot terminal, each of the plurality of tool arms comprises a base joint and a third parallelogram structure formed by articulated links,
the base joint being configured to swing about a first axis so as to drive the surgical instrument to swing about the first axis, the base joint having a proximal end coupled to the adjustment section and a distal end coupled to the third parallelogram structure,
the third parallelogram structure comprising a third proximal link and a third distal link parallel to the third proximal link, the third parallelogram structure having a terminal end coupled to the surgical instrument that is so configured to have an axis parallel to an axis of the distal link so that the third parallelogram structure is able to drive the surgical instrument to pivot about a second axis,
wherein the remote center of motion is defined at an intersection of the first axis and the second axis.

Optionally, in the surgical robot terminal, the terminal further comprises a console for controlling operation of the base, the vertical assembly and/or the robotic arms.

According to the present application, the surgical robot terminal includes a moveable base and a support mechanism disposed between the support end of the vertical assembly and the proximal ends of the setup arms, and the horizontal movability of the base allows the robotic arms (especially their setup arms) to move in an expanded range and enables the terminal to reach any desired position without the use of any additional component supported on a ceiling of an operating room. This allows a simplified overall structure and a reduced mass of the terminal, making it more consistent with the demand for lightweight, structurally simpler medical robots. The employment of the support mechanism not only further expands the robotic arms' movement range but can effectively avoid interference between the robotic arms when they are moving, thus enhancing the terminal's adjustability and operating reliability.

In addition, the movability of the base in combination with the vertical translation of the vertical translational joint in the vertical assembly further allows the remote center of motion (RCM) of each robotic arm and the surgical instrument mounted thereon to more easily access a surgical site in a patient, and the structurally simpler terminal can therefore place the robotic arm to a position more suitable for performing an operation on the surgical site, thereby facilitating the surgical operation. Providing the base with both the drive and guide wheels can decouple speed control from direction control, resulting in both enhanced maneuverability and maneuver accuracy, which are in particular conducive to the overall movement of large equipment such as the surgical robot terminal.

### BRIEF DESCRIPTION OF THE DRAWINGS

Fig. 1 is a principal view of a surgical robot terminal according to an embodiment of the present application.
Fig. 2 schematically illustrates the adjustment to distribution of degrees of freedom of a surgical robot terminal according to an embodiment of the present application.
Fig. 3 shows an enlarged schematic view of robotic arms in a surgical robot terminal according to an embodiment of the present application.
Fig. 4 schematically illustrates the structure of a surgical robot terminal with two arm supports according to an embodiment of the present application.
Fig. 5 is a perspective view of the surgical robot terminal of Fig. 4 in which robotic arms are moving away from a vertical assembly.
Fig. 6 schematically illustrates the structure of a surgical robot terminal with one primary arm support and two secondary arm supports according to an embodiment of the present application.

In these figures,
1 denotes a base; 11, a wheel set; 111, a drive wheel; 112, a guide wheel; 101 and 102, in-plane translational degrees of freedom; 103, a curved movement trajectory;
2, a vertical assembly; 21, a vertical translational joint; 201, a vertical translational degree of freedom; 202, a support end; 203, a base end;
3, a robotic arm; 31, a setup arm; 311, a first rotary joint; 312, a first horizontal translational joint; 313, a swinging joint; 314, a second rotary joint; 301, a first rotational degree of freedom; 302, a first horizontal translational degree of freedom; 303, a swinging degree of freedom; 304, a second rotational degree of freedom; 32, a tool arm; 321, a base joint; 322a, a first parallelogram sub-structure; 322b, a second parallelogram sub-structure; 323, a telescopic joint;
41a, a first arm support; 41b, a second arm support; 42, a primary arm support; 43, a secondary arm support; 401, a third rotational degree of freedom; and
RC, a remote center of motion (RCM).

### DETAILED DESCRIPTION

Specific embodiments of the proposed surgical robot terminal will be described in greater detail below with reference to the accompanying drawings. Features and advantages of the application will be more apparent from the following detailed description, and from the appended claims. Note that the figures are provided in a very simplified form not necessarily presented to scale, with their only intention to facilitate convenience and clarity in explaining the embodiments.

Throughout this specification, a "proximal end" of an object is meant to refer to an end thereof located farther away from a remote center (RC), also called a remote center of motion (RCM), and closer to a vertical assembly, while a "distal end" or "terminal end" thereof is meant to refer to an end closer to the RCM and farther away from the vertical assembly.

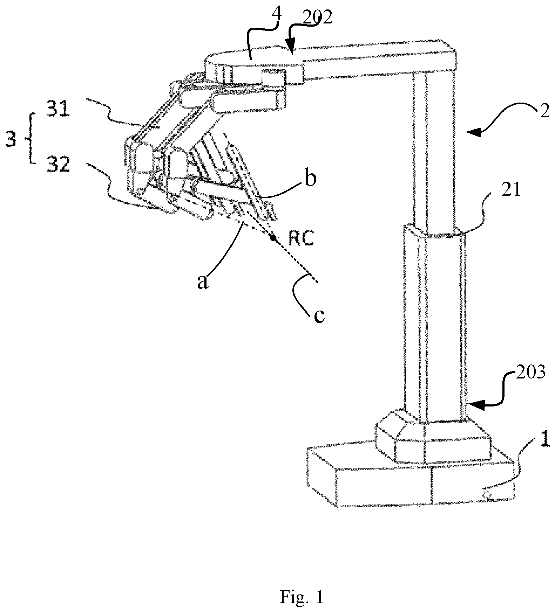

Reference is now made to Fig. 1, a principal view of a surgical robot terminal according to an embodiment of the present application. As shown in Fig. 1, the terminal includes a movable base 1, a vertical assembly 2, a support mechanism (not shown in Fig. 1) and multiple robotic arms 3. The vertical assembly has a base end 203 secured to the base 1 and a support end 202 located above the base 1 and rotatably coupled to the support mechanism. Each robotic arm 3 includes a setup arm 31 and a tool arm 32. A proximal end of the setup arm 31 is coupled to the support mechanism, and a distal end thereof is coupled to the tool arm 32. A distal end of the tool arm 32 is coupled to a surgical instrument. The tool arm 32 is configured to drive the surgical instrument to pivot about a remote center (RC) of motion, while the setup arm 31 is adapted to adjust a spatial position of the RCM. The vertical assembly 2 includes a vertical translational joint 21 for adjusting a height of the RCM relative to the base 1. The support mechanism may include at least two arm supports, to each of which one or more of the robotic arms 3 may be attached, in order to avoid interference between the robotic arms 3. The base 1 may be provided at the bottom with at least two wheel sets each including a drive wheel and a guide wheel respectively configured for movement speed control and movement direction control, thereby facilitating movement of the base 1. A more detailed description of the support mechanism and the wheel sets will be set forth below.

One of the primary objectives of the surgical robot terminal is to allow an arrival of the RCM of the robotic arm 3 at a desired position suitable for the surgical instrument detachably coupled thereto to insert into a patient through an incision made around the RCM to arrive at a target surgical site. This requires a sufficiently wide adjustment range of the surgical robot terminal. On the other hand, the adjustment and tool arms are required to minimize their mutual interference before and during use in a surgical operation. In the present embodiment, the base 1 is moveable in all directions, while the vertical assembly 2 can move up and down. The combination of base 1 and the vertical assembly 2 allows each robotic arm 3 to more easily approach the patient. By providing the support mechanism, interference between the multiple robotic arms 3 can be avoided, resulting in improved adjustment capabilities of the setup arms. In each of the robotic arms 3, the setup arm 31 is capable of performing spatial movement, so when the surgical robot terminal is near the patient, the setup arm 31 can be adjusted to cause an arrival of the RC at a desired position suitable for the surgical instrument to arrive at a surgical site in the patient, and the surgical instrument can be subsequently driven by the tool arm 32 to pivot about the RC so as to accomplish a particular surgical operation.

With continued reference to Fig. 1, the vertical assembly 2 may generally resemble the inverted letter "L". Specifically, the vertical assembly 2 may include a vertical translational joint 21, a horizontal beam and a vertical column fixedly connected to a proximal end of the horizontal beam. The distal end of the horizontal beam serves as the support end 202. The support mechanism is rotatably coupled to the support end 202 with a rotation axis extending parallel to the vertical column and perpendicular to a horizontal plane. The vertical translational joint 21 is arranged on the vertical column (corresponding to a longer side of the L-like shape) and configured to vertically adjust the robotic arm 3 so as to change the height of the RC relative to the base 1.

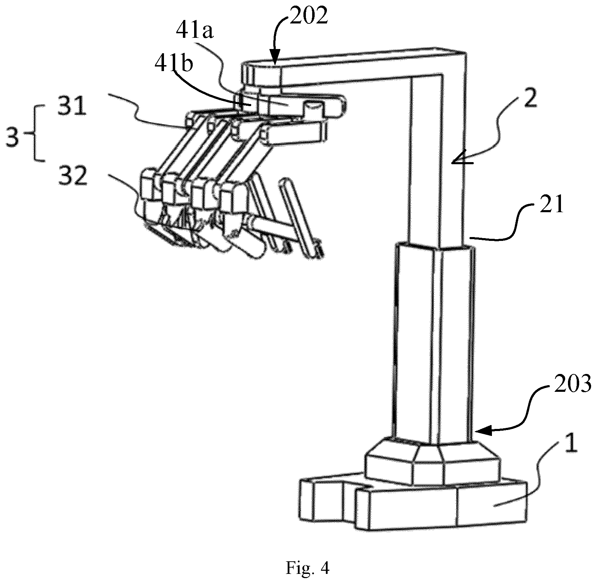

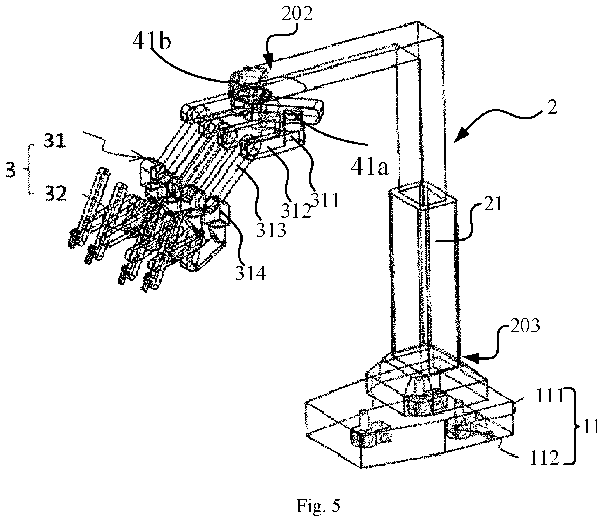

Referring to Figs. 4 and 5, in order to impart movability to the base 1, in the present embodiment, the base 1 essentially includes at least two wheel sets 11 each including a drive wheel 111 and a guide wheel 112, the guide wheel 112 configured for movement direction control, the drive wheel 111 configured for movement speed control. The at least two wheel sets 11 are configured to cooperate to displace the base 1 so as to adjusts a working position of each surgical instrument (relative to the patient). In the present embodiment, the base 1 preferably includes three wheel sets 11, which are arranged into a triangular pattern and motor-driven to enable movement of the base 1. With the adjustability of the three guide wheels 112 and movability of the three drive wheels 111, the surgical robot terminal can overall move along a certain path to approach the patient. Since the base 1 is able to move freely, it can approach the patient at a suitable position compatible with conditions of the patient, and the RC approaches the patient simultaneously with the approaching of the surgical robot. This greatly simplifies the structure of the support end 202 of the vertical assembly 2 and hence overall structure of the surgical robot terminal while reducing the total mass of the surgical robot terminal, making it more consistent with the demand for lightweight, structurally simpler medical robots.

With continued reference to Fig. 5, the robotic arms 3 are oriented away from the vertical assembly 2 (i.e., their terminal ends of the robotic arms 3 extend outwardly). The orientation of each robotic arm 3 is determined by a combination of an angle of rotation and connection mode of the setup arm 31 and that of the tool arm 32. The setup arms 31 and tool arms 32 are rotatably coupled to each other at an angular difference adaptable as required by the surgical operation. That is, the orientation of the robotic arm 3 can be modified as per the actual surgical needs.

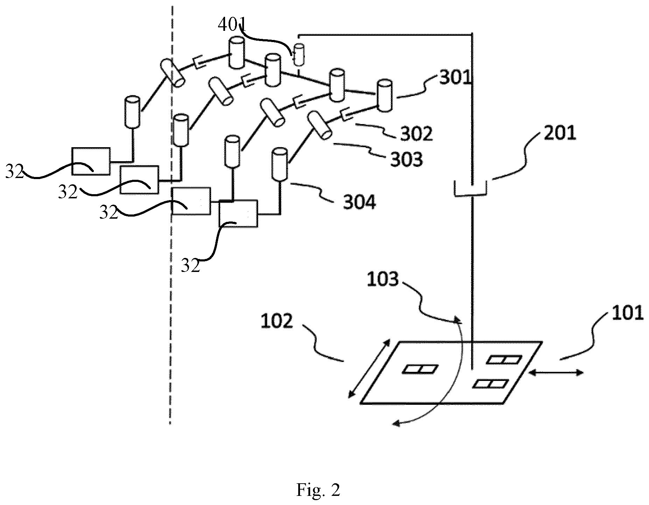

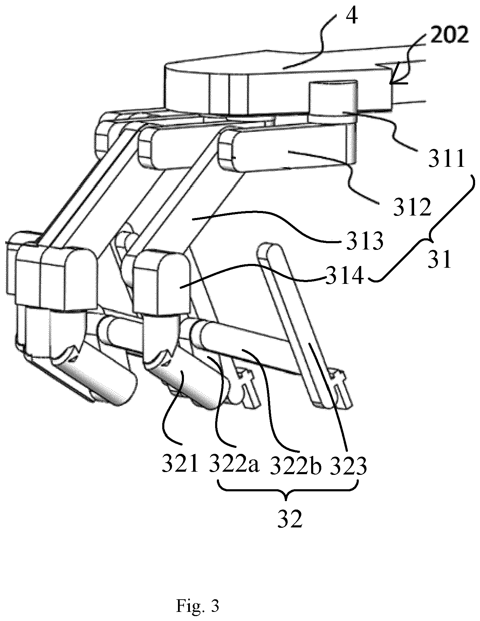

Referring to Figs. 2 to 3, each setup arm 31 includes a first rotary joint 311, a first horizontal translational joint 312, a swinging joint 313 and a second rotary joint 314, which are sequentially connected together in this order, the first horizontal translational joint 312 having an axis of translation (i.e., a movement trajectory or a telescopic direction thereof, or a track in case of the joint 312 being implemented as a slider-track structure) that is perpendicular to an axis of rotation of the first rotary joint 311, the swinging joint 313 having an axis of rotation that is perpendicular to both the axis of translation of the first horizontal translational joint 312 and an axis of rotation of the first rotary joint 311, the second rotary joint 314 having an axis of rotation oriented parallel to the axis of rotation of the first rotary joint 311. The first rotary joint 311 couples the setup arm 31 to the support mechanism, while the second rotary joint 314 couples the setup arm 31 to the tool arm 32. The first rotary joint 311 is able to drive the whole robotic arm 3 to rotate about the axis of rotation of the first rotary joint 311, and the first horizontal translational joint 312 can drive the tool arm 32 to translate horizontally. The swinging joint 313 is configured to swing so as to drive the tool arm 32 to move within a vertical plane. The first and second rotary joints 311, 314 are redundant to each other and configured to enable finer adjustments. These four joints of the setup arm 31 can work together to allow any change of the RC in spatial position.

The present embodiment is not limited to any particular method for making the axis of rotation of the second rotary joint 314 parallel to that of the first rotary joint 311. In a preferred embodiment, the setup arm further includes a connecting rod, a measurement component and a motor communicatively coupled to the measurement component. A proximal end of the connecting rod is coupled to the swinging joint 313, and a distal end thereof is rotatably coupled to the second rotary joint 314. The measurement component is configured to measure a swing angle of the swinging joint 313 in real time. The motor is configured to cause the second rotary joint 314 to rotate relative to the connecting rod in accordance with the swing angle of the swinging joint 313 so as to always maintain the axis of rotation of the second rotary joint 314 parallel to that of the first rotary joint 311. In another preferred embodiment, the swinging joint 313 includes a first parallelogram structure formed by four links articulated together, including a proximal link maintained parallel to the axis of rotation of the first rotary joint 311 and a distal link (that is parallel to the proximal link) that is coupled to the second rotary joint 314 and maintained parallel to the axis of rotation of the second rotary joint 314. In this way, the axis of rotation of the second rotary joint 314 is always maintained parallel to that of the first rotary joint 311.

Preferably, the setup arm 31 further includes a second horizontal translational joint having a proximal end coupled to the support mechanism and a distal end coupled to the first rotary joint 311. Moreover, the second horizontal translational joint has an axis of translation that is perpendicular to the axis of rotation of the first rotary joint 311. In this way, the setup arm 31 has an effectively widened horizontal movement range.

In another embodiment, each setup arm includes a first horizontal translational joint 312, a first rotary joint 311, a swinging joint 313 and a second rotary joint 314, which are sequentially connected together in this order, the first horizontal translational joint 312 having an axis of translation perpendicular to an axis of rotation of the first rotary joint 311, the swinging joint 313 having an axis of rotation perpendicular to the axis of translation of the first horizontal translational joint 312 and perpendicular to the axis of rotation of the first rotary joint 311, the second rotary joint 314 having an axis of rotation oriented parallel to the axis of rotation of the first rotary joint 311. The first horizontal translational joint 312 couples the setup arm 31 to the support mechanism, and the second rotary joint 314 couples the setup arm 31 to the tool arm 32. This arrangement allows full utilization of a space around the support end 202 of the vertical assembly 2.

The tool arm 32 is an RC mechanism capable of causing the surgical instrument coupled thereto to pivot about an RC. For example, the tool arm 32 may have multiple degrees of freedom, such as two degrees of freedom (in this case, the tool arm 32 is able to drive the surgical instrument swing about the RC in both left-and-right and front-and-back directions), three degrees of freedom (in this case, the tool arm 32 is able to drive the surgical instrument to swing about the RC in both left-and-right and front-and-back directions and move up and down).

In the embodiments shown in Figs. 1 and 4, the tool arm 32 has three degrees of freedom so as to be able to drive the surgical instrument to swing about a first axis "a", pivot about a second axis "c" and translate along an axis of translation "b", and the RC is defined at the intersection of the first axis "a", axis of translation "b" and second axis "c" (the three axes are indicated by dashed lines in the figure). Specifically, the tool arm 32 includes a base joint 321, a second parallelogram structure and a telescopic joint 323. The base joint 321 is configured to rotate about the first axis "a" so as to cause the surgical instrument to swing about the first axis "a". A proximal end of the base joint 321 is coupled to the setup arm 31, and a distal end thereof is coupled to the second parallelogram structure. The second parallelogram structure is rotatable and formed by articulated links, including a proximal link and a distal link parallel to the proximal link. A terminal end of the second parallelogram structure is coupled to the surgical instrument through the telescopic joint 323, and the surgical instrument is configured such that an axis thereof is parallel to the distal link. As a result, the second parallelogram structure is able to drive the surgical instrument to pivot about the second axis. In the present embodiment, the second parallelogram structure includes a first parallelogram sub-structure 322a and a second parallelogram sub-structure 322b connected thereto. That is, the second parallelogram structure forms a double-parallelogram structure for driving the surgical instrument to rotate about the second axis "c". The first parallelogram sub-structure 322a includes a first proximal link and a first distal link (both not shown) parallel to the first proximal link. The second parallelogram sub-structure 322b includes a second proximal link and a second distal link (both not shown) parallel thereto. The first distal link may overlap with the second proximal link or the second distal link. The second parallelogram sub-structure 322b has a distal end coupled to the telescopic joint 323, which defines the axis of translation "b" that is parallel to an axis of the second distal link in the second parallelogram sub-structure 322b and extends through the RC. The telescopic joint 323 is detachably coupled to the surgical instrument so as to allow the surgical instrument to translate along the axis of translation "b".

In an alternative embodiment, the tool arm 32 may have only two degrees of freedom so as to be able to drive the surgical instrument to swing about the first axis "a" and pivot about the second axis "c", with the RC being defined at the intersection of the first and second axes "a", "c". In this case, the tool arm 32 includes a base joint 321 and a third parallelogram structure, which is rotatable and formed by articulated links, including a proximal link and a distal link parallel to the proximal link. A terminal end of the third parallelogram structure is detachably coupled to the surgical instrument, and the axis of the surgical instrument is parallel to an axis of the distal link in the third parallelogram structure and extends through the RC. It will be appreciated by those skilled in the art that the tool arm may adopt another structure for driving the surgical instrument to swing about the RC, such as a structure incorporating a base joint and a slider-arcuate track structure coupled to the base joint.

During preliminary preparation for a surgical operation, according to the present embodiment, as a result of movement of the various joints, the setup arm 31 causes the RC of the tool arm 32 to approach a target surgical site in the patient, thereby facilitating the implementation of a more accurate surgical operation. During the operation, all the joints of the setup arm 31 are locked so that the RC is fixed in position, and the surgical instrument detachably coupled to the tool arm 32 is configured to insert into the patient via an incision around the RC to arrive at the target surgical site. The surgical instrument can be driven by the tool arm 32 to swing and pivot within a conical working space with an apex located at the RC. Therefore, adjusting the RC's position through the setup arm 31 allows the surgical instrument mounted on the tool arm 32 to arrive at the target surgical site, and adjusting the individual joints of the tool arm 32 enables the surgical instrument to move within a RC-defined space suitable for a desired particular surgical operation.

Reference is further made to Fig. 2, a diagram illustrating the surgical robot terminal in terms of degree of freedom. The surgical robot terminal's degrees of freedom of adjustment include: in-plane translational degrees of freedom 101, 102 of the base 1 (which together allow the base 1 to move along a curved movement trajectory 103); a vertical translational degree of freedom 201 of the vertical assembly 2; four degrees of freedom of each setup arm 31, which are respectively a first rotational degree of freedom 301, a first horizontal translational degree of freedom 302, a swinging degree of freedom 303 and a second rotational degree of freedom 304; and a third rotational degree of freedom 401 of the support mechanism. The in-plane translational degrees of freedom 101, 102 are enabled by the guide and drive wheels 111, 112 of the base 1. For a desired RC positioned near the dashed line in Fig. 2, the terminal may move around the dashed line as a result of both traveling of the base 1 and upward or downward movement of the vertical assembly 2, so that a corresponding one of the robotic arms 3 can be located at a position closer to the RC and suitable for the robotic arm 3 to arrive at a target surgical site. The four degrees of freedom impart spatial movability to each setup arm 31 and enable the surgical instrument mounted on a terminal end of the corresponding tool arm 32 to more accurately get close to a desired RC. The third rotational degree of freedom 401 of the support mechanism allows each setup arm 31 to move in a wider range without interference between the setup arms 31 and even between the robotic arms 3.

Additionally, each joint in the surgical robot terminal (e.g., those in one or more of the base 1, the vertical assembly 2, the setup arms 31 and the tool arms 32) may be provided with respective brakes configured to control locking or unlocking of these joints. For example, each brake may be in a released state when it is energized by electricity, in which the corresponding joint can move normally, or in a locked state when it is not energized by electricity, in which the joint remains stationary at a particular orientation.

In order to enhance active adjustability of each joint in the surgical robot terminal, each joint may be provided with a motor for allowing its initiative adjustment such as acceleration or deceleration.

Additionally, each joint in the surgical robot terminal may be provided with at least one sensor for sensing status information of the joint, from which a spatial position and orientation of an end effector (not shown) of the corresponding surgical instrument can be derived.

In order to facilitate accurate control of the various components in the surgical robot terminal, the surgical robot terminal may include a console for controlling operation of the base 1, the vertical assembly 2 and/or the robotic arms 3. Specifically, the console is communicatively coupled to the aforementioned sensors, brakes and motors and configured to obtain the status information about the joints from the sensors and lock or unlock the individual joints by means of the respective brakes. In a passive control mode, the console is also configured to facilitate movement of the individual joints by controlling torque outputs of the respective motors.

In one example of the support mechanism, it includes at least two arm supports, each having an upper end coupled to the support end 202 of the vertical assembly 2 and a lower end coupled to at least one setup arm 31. Preferably, each arm support is rotatably coupled to at least one setup arm 31. The arm support can rotate to change a spatial position of the setup arm 31, thereby expanding an adjustment range of the setup arm 31. Further, the support mechanism can adjust spatial positions of the robotic arms 3 before a surgical operation is performed. This can greatly reduce interference between the robotic arms 3 during surgery, thus enhancing the adjustability of the setup arm 31.

In the particular example shown in Fig. 5, the support mechanism includes two arm supports, i.e., a first arm support 41a and a second arm support 41b. Preferably, each of the first and second arm supports 41a, 41b is coupled to both the first rotary joints 311 of two setup arms 31. Moreover, the two setup arms 31 coupled to the first arm support 41a are located on opposite sides thereof, and the two setup arms 31 coupled to the second arm support 41b are likewise located on opposite sides thereof. With this arrangement, interference between the setup arms 31 and even between the corresponding tool arms 32 can be effective avoided.

Similarly, a second horizontal translational joint may be additionally provided between the setup arms 31 and the support mechanism (incorporating the first and second arm supports 41a, 41b). In this case, each setup arm 31 is coupled at the first rotary joint 311 to the support mechanism (i.e., to the first sus-pension disc 41a or the second arm support 41b) via the second horizontal translational joint. This can effectively expand the movement range of the robotic arms 3.

Alternatively, the second horizontal translational joint may be provided between the first arm support 41a or second arm support 41b and the corresponding setup arms 31. In this case, each of the setup arms 31 is coupled at the first rotary joint 311 to the first arm support 41a or second arm support 41b via the second horizontal translational joint, and the aforementioned first horizontal translational joint 312 is omitted, with the swinging joint 313 coupled to the first rotary joint 311. This enables full utilization of the space around the support end 202 of the vertical assembly 2.

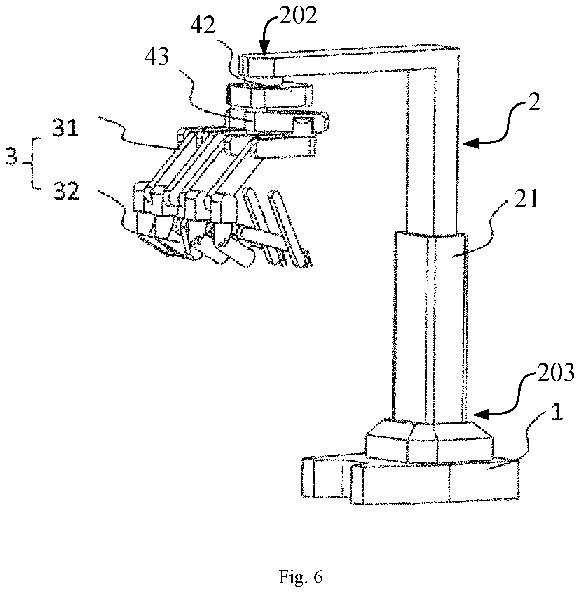

In another example of the support mechanism, with reference to Fig. 6, the surgical robot terminal includes one primary arm support 42 and at least two secondary arm supports 43, the primary arm support 42 having an upper end rotatably coupled to the support end 202 of the vertical assembly 2, each of the at least two secondary arm supports 43 being rotatably coupled to a lower end of the primary arm support 42 and having a lower end coupled to corresponding setup arms 31. The primary arm support 42 can rotate to change spatial positions of the secondary arm supports 43, and in turn, each of the secondary arm supports 43 can rotate to change spatial positions of the setup arms 31 coupled thereto.

In the specific example shown in Fig. 6, the number of the primary arm support 42 is one and the number of the secondary arm supports 43 is two, each of the two secondary arm supports 43 is rotatably coupled at the upper end thereof to the primary arm support 42 so that the secondary arm supports 43 can rotate relative to the primary arm support 42. Moreover, the primary arm support 42 can adjust the position of the secondary arm supports 43, which can in turn adjust the positions of the setup arms 31 that are coupled to them. Each of the secondary arm supports 43 is coupled at the lower end thereof to two setup arms 31 in such a manner that the setup arms 31 are located at opposing sides of a corresponding secondary arm support 43. In other words, the secondary arm support 43 spaces the two setup arms 31 apart from each other. This can avoid interference between the setup arms 31 during use. The movability of the base 1 in combination with that of the primary and secondary arm supports 42, 43 entails a redundant design allowing the RCM of each robotic arm 3 to reach a position suitable for the surgical instrument mounted thereon to perform an operation more accurately on a target surgical site.

As seen from the above description, the surgical robot terminal according to the present embodiment can adjust spatial positions of the setup arms 31 based on the support mechanism, adjust spatial positions of the RCMs based on the setup arms 31, and adjust orientations of the surgical instruments relative to the RCs based on the tool arms 32. Thus, each surgical instrument can be placed at a more suitable position for handling a surgical site, which is consistent with the current clinical needs and helpful in improving surgical accuracy.

Similarly, the second horizontal translational joint may be added between the setup arms 31 and the secondary arm supports 43, with the first rotary joints 311 of the setup arms 31 being coupled to the secondary arm supports 43 via the second horizontal translational joint. This can effectively expand the movement range of the robotic arms 3. Alternatively, the first horizontal translational joints 312 of the setup arm 31 may be omitted, with the first rotary joints 311 being coupled to the respective swinging joints 313. This allows full utilization of the space around the support end 202 of the vertical assembly 2.

The embodiments disclosed herein are described in a progressive manner, with the description of each embodiment focusing on its differences from others. Reference can be made between the embodiments for their identical or similar parts.

To sum up, according to the present application, the surgical robot terminal includes a moveable base and a support mechanism disposed between the support end of the vertical assembly and the proximal ends of the setup arms, and the horizontal movability of the base allows the robotic arms to move in an expanded range and enables the surgical robot terminal to reach any desired position without the use of any additional component supported on a ceiling of an operating room. This allows a simplified overall structure and a reduced mass of the surgical robot terminal, making it more consistent with the demand for lightweight, structurally simpler medical robots. The adjustment of the position of the support mechanism not only further expands the robotic arms' movement range but can effectively avoid interference be-tween the robotic arms when they are moving, thus enhancing the terminal's adjustability and operating reliability.

In addition, the movability of the base in combination with the vertical translation of the vertical translational joint in the vertical assembly further allows each robotic arm to approach the RCM thereof more closely and therefore allows the robotic arm to a position more suitable for performing an operation on the surgical site, thereby facilitating the surgical operation. Providing the base with both the drive and guide wheels can decouple speed control from direction control, resulting in both enhanced maneuverability and maneuver accuracy, which are in particular conducive to the overall movement of large appliances such as the surgical robot terminal.

The description presented above is merely that of a few preferred embodiments of the present application and does not limit the scope thereof in any sense, the scope being defined by the attached independent claim.

## Claims

1. A surgical robot terminal, comprising:
a movable base (1);
a support mechanism comprising at least two arm support;
a vertical assembly (2) having a base (1) end fixedly coupled to the base (1) and a support end (202) located above the base (1) and rotatably coupled to the support mechanism; and
a plurality of robotic arms (3), each comprising a setup arm (31) and a tool arm (32), the setup arm (31) having a proximal end coupled to one of the at least two arm supports and a distal end coupled to the tool arm (32), the tool arm (32) having a distal end coupled to a surgical instrument, the tool arm (32) being configured to drive the surgical instrument to pivot about a remote center of motion, the setup arm (31) being configured to adjust a spatial position of the remote center of motion,
wherein the vertical assembly (2) comprises a vertical translational joint (21) configured to adjust a height of the remote center of motion relative to the base (1), and
wherein the base (1) comprises at least two wheel sets (11), each comprising a drive wheel (111) and a guide wheel (112), the guide wheel (112) being configured for control the movement direction, the drive wheel (111) being configured for control the movement speed, the at least two wheel sets (11) being configured to cooperate to change the position of the base (1) so as to change a horizontal position of the remote center of motion;
wherein each of the arm support has a proximal end coupled to the support end (202) of the vertical assembly (2) and a distal end coupled to at least one of the plurality of setup arms (31);
wherein each of the plurality of setup arms (31) comprises a first horizontal translational joint (312), a first rotary joint (311), a swinging joint (313) and a second rotary joint (314), which are sequentially coupled together successively,
the first horizontal translational joint (312) having an axis of translation that is perpendicular to an axis of rotation of the first rotary joint (311),
the swinging joint (313) having an axis of rotation that is perpendicular to both the axis of translation of the first horizontal translational joint (312) and the axis of rotation of the first rotary joint (311),
the second rotary joint (314) having an axis of rotation oriented parallel to the axis of rotation of the first rotary joint (311),
the first horizontal translational joint (312) coupling the corresponding setup arm (31) to the support mechanism, the second rotary joint (314) coupling the corresponding setup arm (31) to the corresponding tool arm (32).

2. The surgical robot terminal according to claim 1, wherein the support mechanism further comprises a primary arm support having a proximal end rotatably coupled to the support end (202) of the vertical assembly (2), and wherein each of the at least two arm supports has a proximal end rotatably coupled to a distal end of the primary arm support and a distal end coupled to at least one of the plurality of setup arms (31).

3. The surgical robot terminal according to claim 1, wherein each of the plurality of tool arms (32) has three or more degrees of freedom, and thus is able to drive the surgical instrument to at least swing left and right, pivot front and back and move up and down with respect to the remote center of motion.

4. The surgical robot terminal according to claim 3, wherein each of the plurality of tool arms (32) comprises a base joint (321), a telescopic joint (323), and a second parallelogram structure formed by articulated links,
the base joint (321) being configured to swing about a first axis so as to drive the surgical instrument to swing about the first axis, the base joint (321) having a proximal end coupled to the setup arm (31) and a distal end coupled to the second parallelogram structure,
the second parallelogram structure comprising a second proximal link and a second distal link parallel to the second proximal link, the second parallelogram structure configured to drive the surgical instrument to pivot about a second axis,
the telescopic joint (323) being coupled to the second distal link, the telescopic joint (323) having an axis of translation that is parallel to an axis of the second distal link, the telescopic joint (323) being detachably coupled to the surgical instrument so as to be able to drive the surgical instrument to translate along the axis of translation of the telescopic joint (323),
wherein the remote center of motion is defined at an intersection of the first axis, the second axis and the axis of translation of the telescopic joint (323).

5. The surgical robot terminal according to claim 1, wherein each of the plurality of tool arms (32) has two or more degrees of freedom, and thus is able to drive the surgical instrument to at least swing left and right and pivot front and back with respect to the remote center of motion.

6. The surgical robot terminal according to claim 5, wherein each of the plurality of tool arms (32) comprises a base joint (321) and a third parallelogram structure formed by articulated links,
the base joint (321) being configured to swing about a first axis so as to drive the surgical instrument to swing about the first axis, the base joint (321) having a proximal end coupled to the setup arm (31) and a distal end coupled to the third parallelogram structure,
the third parallelogram structure comprising a third proximal link and a third distal link parallel to the third proximal link, the third parallelogram structure having a terminal end coupled to the surgical instrument that is configured to have an axis parallel to an axis of the distal link so that the third parallelogram structure is able to drive the surgical instrument to pivot about a second axis,
wherein the remote center of motion is defined at an intersection of the first axis and the second axis.

7. The surgical robot terminal according to claim 1, further comprising a console for controlling operation of the base (1), the vertical assembly (2) and/or the robotic arms (3).

## Patentansprüche

1. Chirurgisches Roboterterminal, umfassend:
eine bewegliche Basis (1);
einen Stützmechanismus, der mindestens zwei Armstützen umfasst;
eine vertikale Baugruppe (2) mit einem Ende der Basis (1), das fest mit der Basis (1) verbunden ist, und einem Stützende (202), das über der Basis (1) angeordnet und drehbar mit dem Stützmechanismus verbunden ist; und
eine Vielzahl von Roboterarmen (3), die jeweils einen Aufbauarm (31) und einen Werkzeugarm (32) umfassen, wobei der Aufbauarm (31) ein proximales Ende aufweist, das mit einer der mindestens zwei Armstützen gekoppelt ist, und ein distales Ende, das mit dem Werkzeugarm (32) gekoppelt ist, wobei der Werkzeugarm (32) ein distales Ende aufweist, das mit einem chirurgischen Instrument gekoppelt ist, wobei der Werkzeugarm (32) ausgebildet ist, das chirurgische Instrument zum Schwenken um einen entfernten Bewegungspunkt anzutreiben, wobei der Aufbauarm (31) ausgebildet ist, eine räumliche Position des entfernten Bewegungszentrums anzupassen,
wobei die vertikale Baugruppe (2) ein vertikales Translationsgelenk (21) umfasst, das ausgebildet ist, die Höhe des entfernten Bewegungszentrums relativ zu der Basis (1) zu verstellen, und
wobei die Basis (1) mindestens zwei Radsätze (11) umfasst, die jeweils ein Antriebsrad (111) und ein Führungsrad (112) umfassen, wobei das Führungsrad (112) zur Steuerung der Bewegungsrichtung ausgebildet ist, wobei das Antriebsrad (111) zur Steuerung der Bewegungsgeschwindigkeit ausgebildet ist, wobei die mindestens zwei Radsätze (11) ausgebildet sind, zusammenzuwirken, um die Position der Basis (1) zu ändern, um eine horizontale Position des entfernten Bewegungszentrums zu ändern;
wobei jeder der Armstützen ein proximales Ende, das mit dem Stützende (202) der vertikalen Baugruppe (2) verbunden ist, und ein distales Ende aufweist, das mit mindestens einem der Vielzahl von Aufbauarmen (31) verbunden ist;
wobei jeder der Vielzahl von Aufbauarmen (31) ein erstes horizontales Translationsgelenk (312), ein erstes Drehgelenk (311), ein Schwenkgelenk (313) und ein zweites Drehgelenk (314) umfasst, die nacheinander sequentiell gekoppelt sind,
wobei das erste horizontale Translationsgelenk (312) eine Translationsachse aufweist, die zu einer Drehachse des ersten Drehgelenks (311) senkrecht ist,
wobei das Schwenkgelenk (313) eine Drehachse aufweist, die sowohl zu der Translationsachse des ersten horizontalen Translationsgelenks (312) als auch zu der Rotationsachse des ersten Drehgelenks (311) senkrecht ist,
wobei das zweite Drehgelenk (314) eine Drehachse aufweist, die parallel zu der Drehachse des ersten Drehgelenks (311) ausgerichtet ist,
wobei das erste horizontale Translationsgelenk (312) den entsprechenden Aufbauarm (31) an den Stützmechanismus koppelt, wobei das zweite Drehgelenk (314) den entsprechenden Aufbauarm (31) an den entsprechenden Werkzeugarm (32) koppelt.

2. Chirurgisches Roboterterminal nach Anspruch 1, wobei der Stützmechanismus ferner eine Primärarmstütze mit einem proximalen Ende umfasst, das drehbar mit dem Stützende (202) der vertikalen Baugruppe (2) verbunden ist, und wobei jede der mindestens zwei Armstützen ein proximales Ende, das drehbar mit einem distalen Ende der Primärarmstütze gekoppelt ist, und ein distales Ende aufweist, das mit mindestens einem der Vielzahl von Aufbauarmen (31) gekoppelt ist.

3. Chirurgisches Roboterterminal nach Anspruch 1, wobei jeder der Vielzahl von Werkzeugarmen (32) drei oder mehr Freiheitsgrade aufweist, und somit in der Lage ist, das chirurgische Instrument zumindest zum Schwenken nach links und rechts, zum Schwenken nach vorne und hinten und zum Auf- und Abbewegen bezüglich des entfernten Bewegungszentrums anzutreiben.

4. Chirurgisches Roboterterminal nach Anspruch 3, wobei jeder der Vielzahl von Werkzeugarmen (32) ein Basisgelenk (321), ein Teleskopgelenk (323) und eine zweite Parallelogrammstruktur umfasst, die durch Gelenkverbindungen gebildet wird,
wobei das Basisgelenk (321) ausgebildet ist, um eine erste Achse zu schwenken, um das chirurgische Instrument zum Schwenken um die erste Achse anzutreiben, wobei das Basisgelenk (321) ein proximales Ende, das mit dem Aufbauarm (31) gekoppelt ist, und ein distales Ende aufweist, das mit der zweiten Parallelogrammstruktur gekoppelt ist,
wobei die zweite Parallelogrammstruktur ein zweites proximales Glied und ein zum zweiten proximalen Glied paralleles zweites distales Glied umfasst, wobei die zweite Parallelogrammstruktur ausgebildet ist, das chirurgische Instrument zum Schwenken um eine zweite Achse anzutreiben,
wobei das Teleskopgelenk (323) mit der zweiten distalen Verbindung gekoppelt ist, wobei das Teleskopgelenk (323) eine Translationsachse aufweist, die parallel zu einer Achse der zweiten distalen Verbindung ist, und wobei das Teleskopgelenk (323) lösbar mit dem chirurgischen Instrument gekoppelt ist, um in der Lage zu sein, das chirurgische Instrument zur Translation entlang der Translationsachse des Teleskopgelenks (323) anzutreiben,
wobei das entfernte Bewegungszentrum an einem Schnittpunkt der ersten Achse, der zweiten Achse und der Translationsachse des Teleskopgelenks (323) definiert ist.

5. Chirurgisches Roboterterminal nach Anspruch 1, wobei jeder der Vielzahl von Werkzeugarmen (32) zwei oder mehr Freiheitsgrade aufweist, und somit in der Lage ist, das chirurgische Instrument zumindest zum Schwenken nach links und rechts und zum Schwenken nach vorne und hinten bezüglich des entfernten Bewegungszentrums anzutreiben.

6. Chirurgisches Roboterterminal nach Anspruch 5, wobei jeder der Vielzahl von Werkzeugarmen (32) ein Basisgelenk (321) und eine dritte Parallelogrammstruktur umfasst, die durch Gelenkverbindungen gebildet wird,
wobei das Basisgelenk (321) ausgebildet ist, um eine erste Achse zu schwenken, um das chirurgische Instrument zum Schwenken um die erste Achse anzutreiben, wobei das Basisgelenk (321) ein proximales Ende, das mit dem Aufbauarm (31) gekoppelt ist, und ein distales Ende aufweist, das mit der dritten Parallelogrammstruktur gekoppelt ist,
wobei die dritte Parallelogrammstruktur ein drittes proximales Glied und ein zu dem dritten proximalen Glied paralleles drittes distales Glied umfasst, wobei die dritte Parallelogrammstruktur ein mit dem chirurgischen Instrument gekoppeltes Anschlussende aufweist, das ausgebildet ist, eine zu einer Achse des distalen Verbindungsglieds parallele Achse aufzuweisen, so dass die dritte Parallelogrammstruktur in der Lage ist, das chirurgische Instrument zum Schwenken um eine zweite Achse anzutreiben,
wobei das entfernte Bewegungszentrum an einem Schnittpunkt der ersten Achse und der zweiten Achse definiert ist.

7. Chirurgisches Roboterterminal nach Anspruch 1, das ferner eine Konsole zur Steuerung des Betriebs der Basis (1), der vertikalen Baugruppe (2) und/oder der Roboterarme (3) umfasst.

## Revendications

1. Un terminal de robot chirurgical, comprenant:
une base mobile (1);
un mécanisme de support comprenant au moins deux supports de bras;
un ensemble vertical (2) ayant une extrémité de base (1) couplée de manière fixe à la base (1) et une extrémité de support (202) située au-dessus de la base (1) et couplée de manière rotative au mécanisme de support; et
une pluralité de bras robotiques (3), comprenant chacun un bras de réglage (31) et un bras d'outil (32), le bras de réglage (31) ayant une extrémité proximale couplée à l'un des au moins deux supports de bras et une extrémité distale couplée au bras d'outil (32), le bras d'outil (32) ayant une extrémité distale couplée à un instrument chirurgical, le bras d'outil (32) étant configuré pour entraîner l'instrument chirurgical à pivoter autour d'un centre de mouvement distant, le bras de réglage (31) étant configuré pour ajuster une position spatiale du centre de mouvement distant,
dans lequel l'ensemble vertical (2) comprend une articulation de translation verticale (21) configurée pour ajuster une hauteur du centre de mouvement distant par rapport à la base (1), et
dans lequel la base (1) comprend au moins deux ensembles de roues (11), comprenant chacun une roue motrice (111) et une roue de guidage (112), la roue de guidage (112) étant configurée pour commander le sens de déplacement, la roue motrice (111) étant configuré pour contrôler la vitesse de déplacement, les au moins deux ensembles de roues (11) étant configurés pour coopérer pour modifier la position de la base (1) de manière à modifier une position horizontale du centre de déplacement distant;
dans lequel chacun des bras le support a une extrémité proximale couplée à l' extrémité de support (202) de l'ensemble vertical (2) et une extrémité distale couplée à au moins l'un de la pluralité de bras de montage (31);
dans lequel chacun de la pluralité de bras de réglage (31) comprend une première articulation de translation horizontale (312), une première articulation rotative (311), une articulation oscillante (313) et une seconde articulation rotative (314), qui sont séquentiellement accouplées successivement,
la première articulation de translation horizontale (312) ayant un axe de translation perpendiculaire à un axe de rotation de la première articulation rotative (311),
l'articulation oscillante (313) ayant un axe de rotation perpendiculaire à la fois à l'axe de translation de la première articulation de translation horizontale (312) et à l'axe de rotation de la première articulation rotative (311),
le deuxième joint tournant (314) ayant un axe de rotation orienté parallèlement à l'axe de rotation du premier joint tournant (311),
la première articulation de translation horizontale (312) couplant le montage correspondant bras (31) au mécanisme de support, le deuxième joint tournant (314) couplant le configuration correspondante bras (31) au bras d'outil correspondant (32).

2. Terminal de robot chirurgical selon la revendication 1, dans lequel le mécanisme de support comprend en outre un bras principal support ayant une extrémité proximale couplée de manière rotative à l' extrémité de support (202) de l'ensemble vertical (2), et dans lequel chacun des au moins deux bras les supports ont une extrémité proximale couplé de manière rotative à une extrémité distale du bras primaire support et une extrémité distale couplée à au moins l'un de la pluralité de bras de montage (31).

3. Terminal de robot chirurgical selon la revendication 1, dans lequel chacun de la pluralité de bras d'outil (32) a trois degrés de liberté ou plus, et est ainsi capable d' entraîner l'instrument chirurgical pour qu'il oscille au moins vers la gauche et la droite, pivote vers l'avant et vers l'arrière et se déplacer de haut en bas par rapport au centre de mouvement distant.

4. Terminal de robot chirurgical selon la revendication 3, dans lequel chacun de la pluralité de bras d'outil (32) comprend une articulation de base (321), une articulation télescopique (323) et une seconde structure en parallélogramme formée par des liaisons articulées,
l'articulation de base (321) étant configurée pour osciller autour d'un premier axe de façon à entraîner l' instrument chirurgical pour osciller autour du premier axe, l'articulation de base (321) ayant une extrémité proximale couplée au montage bras (31) et une extrémité distale couplée à la seconde structure en parallélogramme,
la deuxième structure en parallélogramme comprenant un deuxième lien proximal et un deuxième lien distal parallèle au deuxième lien proximal, la deuxième structure en parallélogramme configuré pour entraîner l'instrument chirurgical à pivoter autour d'un second axe,
l'articulation télescopique (323) étant couplée au deuxième lien distal, l'articulation télescopique (323) ayant un axe de translation qui est parallèle à un axe du deuxième lien distal, l'articulation télescopique (323) étant couplée de manière amovible à l'instrument chirurgical de manière à pouvoir entraîner l'instrument chirurgical en translation selon l'axe de translation de l'articulation télescopique (323),
dans lequel le centre de mouvement distant est défini à une intersection du premier axe, du deuxième axe et de l'axe de translation de l'articulation télescopique (323).

5. Terminal de robot chirurgical selon la revendication 1, dans lequel chacun de la pluralité de bras d'outil (32) a deux degrés de liberté ou plus, et est ainsi capable d' entraîner l'instrument chirurgical pour qu'il oscille au moins vers la gauche et la droite et pivote vers l'avant et vers l'arrière avec par rapport au centre distant du mouvement.

6. Terminal de robot chirurgical selon la revendication 5, dans lequel chacun de la pluralité de bras d'outil (32) comprend une articulation de base (321) et une troisième structure en parallélogramme formée par des liaisons articulées,
l'articulation de base (321) étant configurée pour osciller autour d'un premier axe de façon à entraîner l'instrument chirurgical pour osciller autour du premier axe, l'articulation de base (321) ayant une extrémité proximale couplée au montage bras (31) et une extrémité distale couplée à la troisième structure en parallélogramme,
la troisième structure en parallélogramme comprenant un troisième lien proximal et un troisième lien distal parallèle au troisième lien proximal, la troisième structure en parallélogramme ayant une extrémité terminale couplée à l'instrument chirurgical qui est configurée pour avoir un axe parallèle à un axe de la liaison distale de sorte que la troisième structure en parallélogramme est apte à entraîner l'instrument chirurgical en pivotement autour d'un second axe,
dans lequel le centre de mouvement distant est défini à une intersection du premier axe et du deuxième axe.

7. Terminal de robot chirurgical selon la revendication 1, comprenant en outre une console pour commander le fonctionnement de la base (1), de l'ensemble vertical (2) et/ou des bras robotiques (3).
